# EUROPEAN PATENT APPLICATION

(11) **EP 4 005 983 A1**
(43) Date of publication of application: **01.06.2022**
(21) Application number: 20210308.1
(22) Date of filing: 27.11.2020
(51) Int. Cl.: C02F 1/78, C02F 1/467, A61L 2/20, A47J 31/60

(54) **DISINFECTION AND STERILIZATION DEVICE OF BEVERAGE MAKER**

(71) Applicant: Yantai United Ozonetec Corporation, Yantai, Shandong (CN)
(72) Inventor: HSU, MING-YUNG, Shandong (CN); HSU, WEI-CHUN, Shandong (CN)
(74) Representative: Zeitler Volpert Kandlbinder Patentanwälte Partnerschaft mbB

(57) **Abstract**

A disinfection and sterilization device 9 of a beverage maker 1 is disclosed. The beverage maker 1 has an operation panel 2, a beverage outlet 3, and a water receiving tank 4 under the beverage outlet. The beverage maker is provided with a power cord 5 and a feed-water pipe 7. One end of the feed-water pipe 7 is connected to the beverage maker, and the other end of the feed-water pipe is connected to an ozone water producing device 9. The ozone water producing device 9 is connected with a water supply apparatus through a water supply pipe 8. The electrolytic ozone water generator is directly connected to the water supply pipe of the beverage maker. The ozone water producing device works to generate ozone gas microbubbles that are quickly dissolved and mixed in the water to generate ozone water. The internal pipe and the beverage outlet of the beverage maker through which the ozone water flows are sterilized.

## Description

### FIELD OF THE INVENTION

The present invention relates to a disinfection and sterilization device, and more particularly to a disinfection and sterilization device applied to a beverage maker.

### BACKGROUND OF THE INVENTION

Since the water used for a beverage maker enters the beverage maker after being processed by a program, it is prone to contamination and breeding of bacteria and mold. Because the beverage is added with nutrients such as sugar and milk, after a night of inactivity especially in the hot season, a large number of bacteria will breed in the internal pipe and the outlet of the beverage maker. Some well-known fast food restaurants provide beverages with excessive bacteria that are often reported by media. This not only seriously affects the users' health, but also brings serious troubles to the business activities and goodwill of the catering services industry.

Therefore, the structure of the existing beverage maker needs to be improved. The inventor believes that ozone can be applied to this field as an efficient and residue-free sterilization technology.

### SUMMARY OF THE INVENTION

In order to improve the aforementioned problem that the internal pipe and the outlet of the conventional beverage maker are prone to breed a large number of bacteria, the primary object of the present invention is to overcome the above-mentioned shortcomings of the prior art and provide a disinfection and sterilization device of a beverage maker.

In order to achieve the above object, a disinfection and sterilization device of a beverage maker is provided. The beverage maker has an operation panel, a beverage outlet, and a water receiving tank under the beverage outlet. The beverage maker is provided with a power cord and a feed-water pipe. One end of the feed-water pipe is connected to the beverage maker, and the other end of the feed-water pipe is connected to an ozone water producing device. The ozone water producing device is connected with a water supply apparatus through a water supply pipe.

Furthermore, the ozone water production device includes an ozone water generator outer casing, an ozone water generator cover, and a bottom casing. The ozone water generator cover is arranged in the ozone water generator outer casing. The ozone water generator outer casing and the ozone water generator cover are tightly connected. The ozone water generator cover is tightly connected to the bottom casing. The bottom casing is provided with a power port and a control port. A control board is provided in the bottom casing. The control board is connected to the control port through a wire. The control board is connected to the power port through a wire. The ozone water generator outer casing is provided with a water inlet and a water outlet. The water inlet is connected to the water supply apparatus through the water supply pipe. The water outlet is connected to the beverage maker through the feed-water pipe. The ozone water generator cover is provided with an ozone water generator anode plate and an ozone water generator cathode plate. The ozone water generator anode plate and the ozone water generator cathode plate are connected to the control board through a wire. The power port is connected to the beverage maker through a wire. The control port is connected to the beverage maker through a wire.

The beneficial effect provided by the present invention is that the present invention adopts an electrolytic ozone water generator. The electrolytic ozone water generator has a simple structure and low cost. The electrolytic ozone water generator is placed outside the casing of the beverage maker and directly connected to the water supply pipe of the beverage maker, which is convenient for replacement and maintenance. The ozone water producing device works to generate ozone gas microbubbles. At the moment when ozone gas microbubbles are generated, the ozone gas microbubbles are quickly dissolved and mixed in the water to directly generate ozone water. The internal pipe and the beverage outlet of the beverage maker through which the ozone water flows are system wide uniformly sterilized.

### BRIEF DESCRIPTION OF THE DRAWINGS

FIG. 1 is a structural schematic view of the present invention; and
FIG. 2 is a structural schematic view of an electrolytic ozone water generator of the present invention.

### DETAILED DESCRIPTION OF THE PREFERRED EMBODIMENTS

Embodiments of the present invention will now be described, by way of example only, with reference to the accompanying drawings.

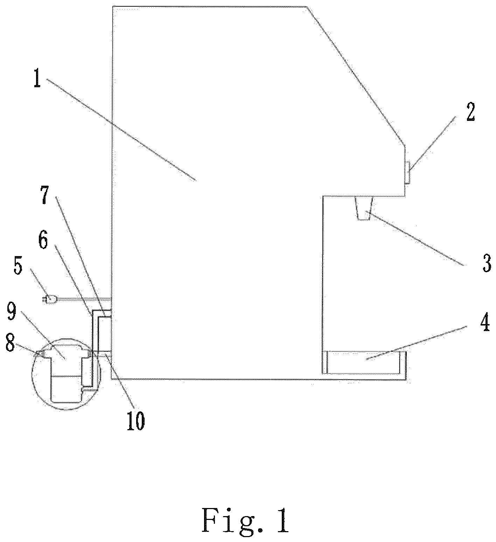

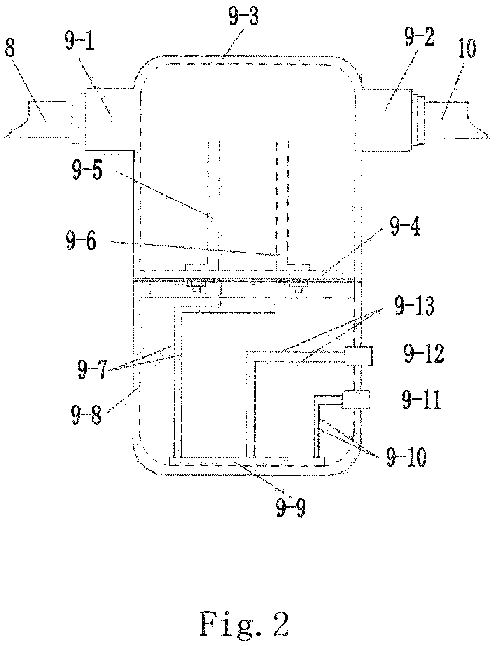

As shown in FIG. 1 and FIG. 2, the present invention discloses a disinfection and sterilization device of a beverage maker 1. The beverage maker 1 has an operation panel 2, a beverage outlet 3 and a water receiving tank 4 under the beverage outlet 3. The beverage maker 1 is provided with a power cord 5 and a feed-water pipe 10. One end of the feed-water pipe 10 is connected to the beverage maker 1, and the other end of the feed-water pipe 10 is connected to an ozone water producing device 9. The ozone water producing device 9 is connected with a water supply apparatus through a water supply pipe 8.

The ozone water production device 9 includes an ozone water generator outer casing 9-3, an ozone water generator cover 9-4, and a bottom casing 9-8. The ozone water generator cover 9-4 is arranged in the ozone water generator outer casing 9-3. The ozone water generator outer casing 9-3 and the ozone water generator cover 9-4 are assembled to form an enclosed space. The ozone water generator cover 9-4 is inserted into the bottom casing 9-8 and attached to the bottom casing 9-8. The bottom casing 9-8 is provided with a power port 9-12 and a control port 9-11. A control board 9-9 is provided in the bottom casing 9-8. The control board 9-9 is connected to the control port 9-11 through a fourth wire 9-10. The control board 9-9 is connected to the power port 9-12 through a fifth wire 9-13. The ozone water generator outer casing 9-3 is provided with a water inlet 9-1 and a water outlet 9-2. The water inlet 9-1 is connected to the water supply apparatus through the water supply pipe 8. The water outlet 9-2 is connected to the beverage maker 1 through the feed-water pipe 10. The ozone water generator cover 9-4 is provided with an ozone water generator anode plate 9-5 and an ozone water generator cathode plate 9-6. The ozone water generator anode plate 9-5 and the ozone water generator cathode plate 9-6 are connected to the control board 9-9 through a third wire 9-7. The power port 9-12 is connected to the beverage maker 1 through a first wire 6. The control port 9-11 is connected to the beverage maker 1 through a second wire 7. The beverage maker 1 supplies power to the ozone water producing device 9 through the first wire 6 and the power port 9-12.

When the beverage maker needs to be sterilized, the disinfection button on the operation panel 2 of the beverage maker 1 is pressed. The beverage maker 1 provides a signal to the control board 9-9 through the second wire 7 and the control port 9-11. The control board 9-9 supplies power to the ozone water generator anode plate 9-5 and the ozone water generator cathode plate 9-6. The ozone water producing device 9 works to generate ozone gas microbubbles. At the moment when ozone gas microbubbles are generated, the ozone gas microbubbles are quickly dissolved and mixed in the water to directly generate ozone water. After working for a period of time, preferably 3 minutes, the water outlet valve of the beverage maker opens. Drinking water enters the inside of the ozone water generator through the water supply pipe 8 and the water inlet 9-1, and the generated ozone water flows into the beverage maker 1 through the water outlet 9-2 and the feed-water pipe 10. The ozone water flows out through the internal pipe of the beverage maker and the beverage outlet 3 and falls into the water receiving tank 4. The internal pipe and the beverage outlet of the beverage maker through which the ozone water flows are system wide uniformly sterilized.

Although particular embodiments of the present invention have been described in detail for purposes of illustration, various modifications and enhancements may be made without departing from the spirit and scope of the present invention. Accordingly, the present invention is not to be limited except as by the appended claims.

## Claims

1. A disinfection and sterilization device of a beverage maker (1), the beverage maker (1) having an operation panel (2), a beverage outlet (3), and a water receiving tank (4) under the beverage outlet (3), the beverage maker (1) being provided with a power cord (5) and a feed-water pipe (10); **characterized in that**: one end of the feed-water pipe (10) is connected to the beverage maker (1), another end of the feed-water pipe (10) is connected to an ozone water producing device (9), the ozone water producing device (9) is connected with a water supply apparatus through a water supply pipe (8).

2. The disinfection and sterilization device of the beverage maker (1) as claimed in claim 1, wherein the ozone water production device (9) includes an ozone water generator outer casing (9-3), an ozone water generator cover (9-4), and a bottom casing (9-8).

3. The disinfection and sterilization device of the beverage maker (1) as claimed in claim 2, wherein the ozone water generator cover (9-4) is arranged in the ozone water generator outer casing (9-3), the ozone water generator outer casing (9-3) and the ozone water generator cover (9-4) are tightly connected, and the ozone water generator cover (9-4) is tightly connected to the bottom casing (9-8).

4. The disinfection and sterilization device of the beverage maker (1) as claimed in claim 3, wherein the bottom casing (9-8) is provided with a power port (9-12) and a control port (9-11), a control board (9-9) is provided in the bottom casing (9-8), the control board (9-9) is connected to the control port (9-11) through a fourth wire (9-10), and the control board (9-9) is connected to the power port (9-12) through a fifth wire (9-13).

5. The disinfection and sterilization device of the beverage maker (1) as claimed in claim 4, wherein the ozone water generator outer casing (9-3) is provided with a water inlet (9-1) and a water outlet (9-2), the water inlet (9-1) is connected to the water supply apparatus through the water supply pipe (8), the water outlet (9-2) is connected to the beverage maker (1) through the feed-water pipe (10).

6. The disinfection and sterilization device of the beverage maker (1) as claimed in claim 5, wherein the ozone water generator cover (9-4) is provided with an ozone water generator anode plate (9-5) and an ozone water generator cathode plate (9-6).

7. The disinfection and sterilization device of the beverage maker (1) as claimed in claim 6, wherein the ozone water generator anode plate (9-5) and the ozone water generator cathode plate (9-6) are connected to the control board (9-9) through a third wire (9-7).

8. The disinfection and sterilization device of the beverage maker (1) as claimed in claim 7, wherein the power port (9-12) is connected to the beverage maker (1) through a first wire (6).

9. The disinfection and sterilization device of the beverage maker (1) as claimed in claim 8, wherein the control port (9-11) is connected to the beverage maker (1) through a second wire (7).
